# EUROPEAN PATENT APPLICATION

(11) **EP 1 440 662 A1**
(43) Date of publication of application: **28.07.2004**
(21) Application number: 04001424.3
(22) Date of filing: 23.01.2004
(51) Int. Cl.: A61B 17/15

(54) **System of motorized guidance for operative instrumentation**

(30) Priority: 27.01.2003 IT MI20030123
(71) Applicant: Medacta International S.A., 6874 Castel San Pietro (TI) (IT)
(72) Inventor: Bernardoni, Massimiliano, 6900 Lugano (CH); Furia, Giovanni, 6702 Claro (CH); Galli, Andrea, 6963 Pragassona (CH); Le Foll, Dominique, 95330 Donot (FR); Orlandini, Luca, 6900 Lugano (CH); Siccardi, Alberto, 6968 Sonvico (CH)
(74) Representative: Incollingo, Italo

(57) **Abstract**

System of motorized guidance for operative instrumentation especially for knee surgery, comprising a cutting guide and means for its movement, characterized by an apparatus of parallel kinematics i.e. of linear movement along at least four axes two by two parallel, with the addition of a further movement of rotation centered on said cutting guide, realizing thereby a total movement with six degrees of freedom.

## Description

The present invention concerns a system of motorized guide for surgical instrumentations, especially for arthroplasty and more particularly for knee surgery.

In a very advantageous and therefore preferred embodiment of the invention, the guide system comprises an apparatus with at least four axes of linear moviment and at least a further rotational axis, said axes bringing about a system with six freedom degrees by exploiting the parallel kinematics.

### Prior Art

In general the usual knee operations are carried out by utilizing a classic mechanical instrumentation which is manually piloted by the surgeon with the risk of imprecisions relative to the pre-operative planning (RX, plates).

At least two inconveniences can be encountered: 1) - the operative planning is per sé little precise and therefore scarsely reliable; 2) - to this intrinsic imprecision of the operative planning must be added the imprecision of the instrumentation positioning.

To obviate the first inconvenience, the use of a surgical navigator is being experienced, which should allow a planning in the course of the intervention (called pre-operative) through a kinematic analysis of the limb to be surgically operated.

This corresponds to a passage from a static analysis to a kinematic analysis. The use of the (virtual) kinematic planning risks to loose its beneficial effects as long as a surgical instrumentation not- automatically (f.i; manually or half-manually) positionable is being used.

In addition to their imprecision susceptibility, the manual operations shows the further inconvenience of requiring very long times of execution.

In the Patent Publication WO 98/40037 (corresp. To US Pat. 6.385.475) describes a process and device for the preoperative determination of the positioning data of endoprosthetic parts in which a respective outer articular point is determinated by way of movement of the bones about a respective outer joint located at the end of the said bones facing away from the central joint.

According to an other Publication (WO 02/36031) the position of a knee prosthesis is determinated with a system comprising means for determining: - the shape of the tibia; - its position relative to the ankle joint; - a high point of the tibial plateau: - the position of the ankle; - the orientation and size of the tibial prosthesis etc. by taking into account several parameters such as the perpendicular to the section plane passing through the centers of the prosthesis and of the ankle joint.

EP 1079756 concerns an interactive computer-assisted surgical system using three-dimensional models of anatomical structures whose positions relative to a surgical tool are registered in real time.

The first object of the present invention is to provide a system which eliminates the Prior Art drawbacks.

An other object of the invention is to provide a system which is particularly efficient at least in terms of precision and duration, for the passage from the virtual planning (surgical navigator) to the relevant execution of the operative intervention.

A further object of the invention is a robot which incorporates in a very compact form, the system components and the relevant means of command and controll f.i. by microprocessor.

The main characteristics of the invention will clearely appear from the embodiments represented in the accompanying drawings in which:
- Figures 1 and 2' are perspective views of the robotizable system according to the invention;
- Figure 2 is a side view of fig. 1; and
- Figure 3 is a cross section view with a plane having as a trace the line X-X in fig. 1.

In a prior Patent Application (N° MI2002A 002218) Applicant has described a "System and Method of orthopedic operations with no additional invasivity", comprising an instrumentation for anchorage to the bone brought about with the aid of a device comprising: - a bracket; - at least one intercondylar pin also of fixation; and - means for the stationary application of said instrumentation.

In this Patent Application it is only fleetingly hinted at the possibility of using a robot to move a surgical instrumentation in particular of a cutting guide.

In the continuation of his researches, Applicant has found and developed a highly performing robotizable system of automated movement.

For illustrative clarity scruple it is convenient to refer (without introducing limitations) to the system according to the above mentioned Patent Application N° M12002A 002218, the description and drawings of which are considered herewith incorporated.

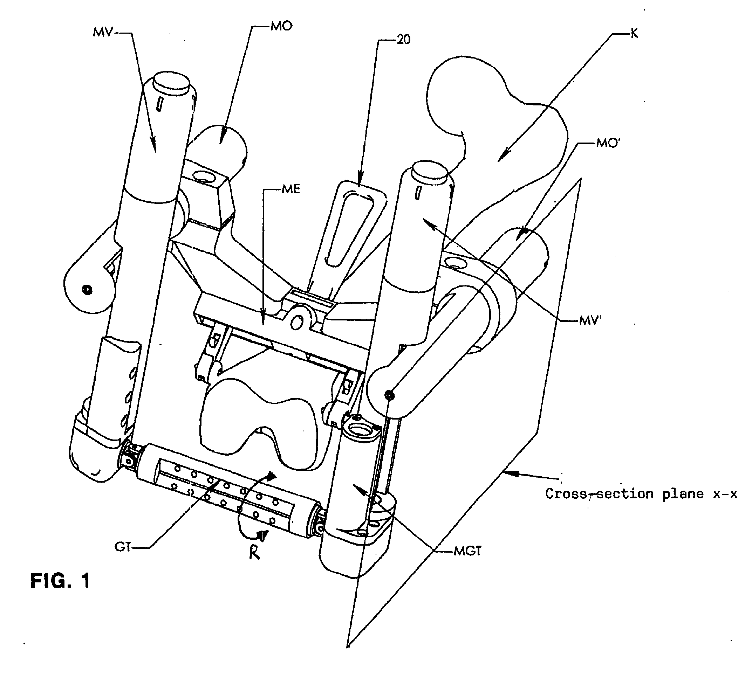

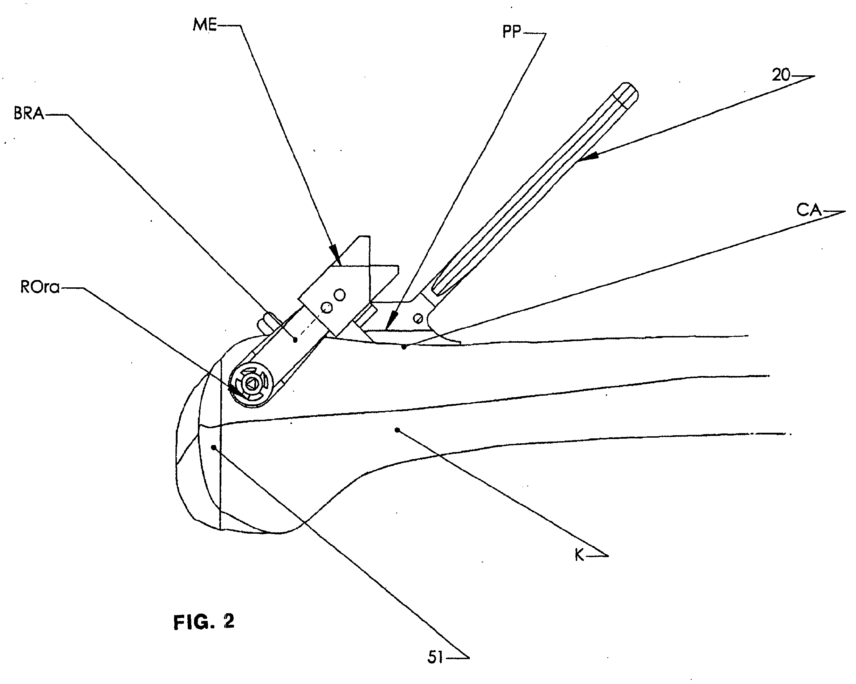

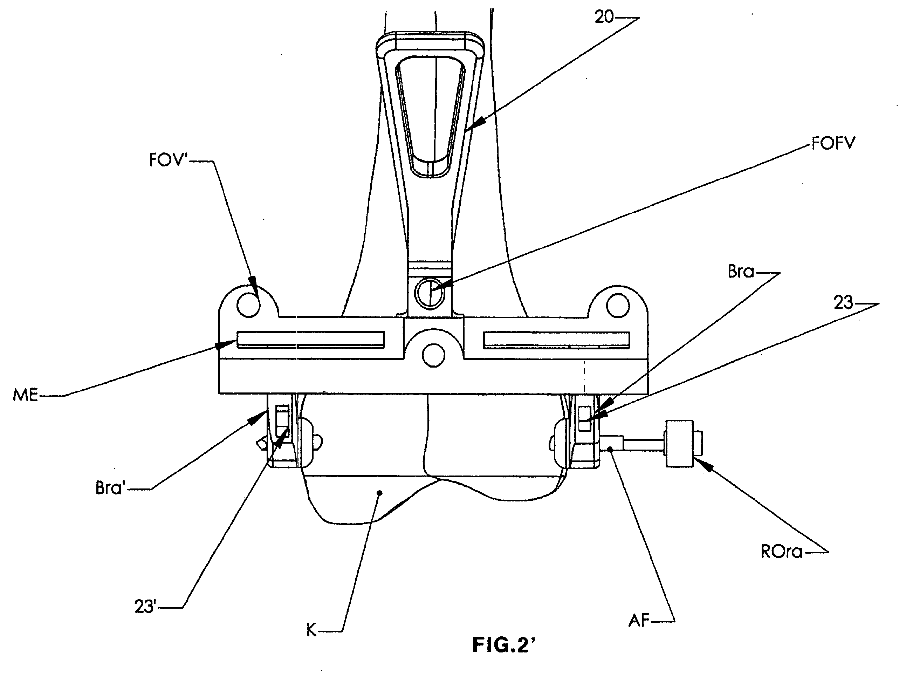

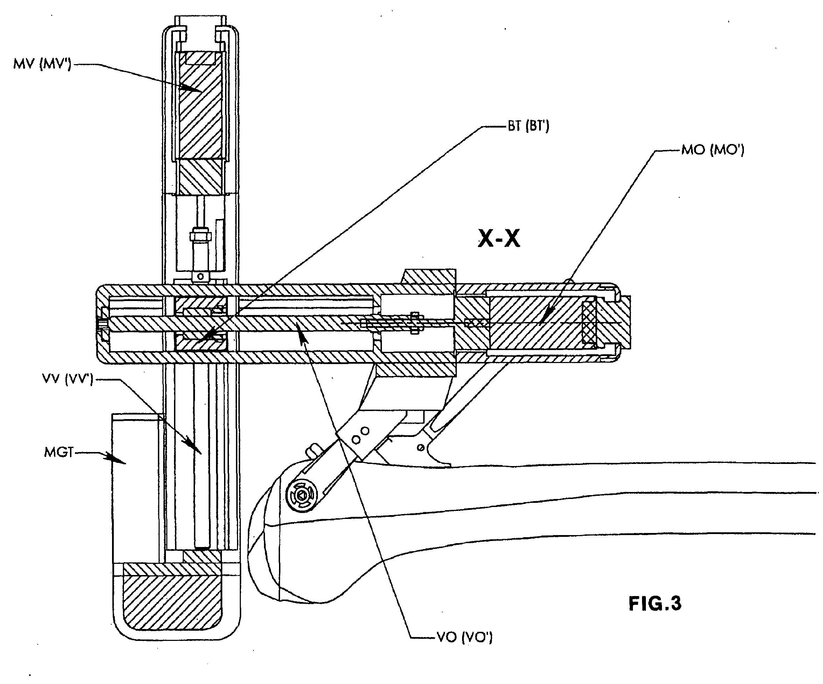

In the perspective view of figures 1 and 2' and in the side view of figure 2, as well as in the view of fig. 3 which is a cross-section with a plane having as trace the line X-X in figure 1, are represented: - a femur K; - an instrumentation for the anchorage to said femur, consisting of: - at least a bracket ME; - at leasat a shovel or grip 20; - at least two arms BRA and BRA' associated to at least a rod AF; - at least a cortical screw FO-FV. For the movement of the cutting means GT are utilized motors MO and MV (for the horizontal respectively vertical movements).

According to a first feature of the invention, a helicoidal screw VO-VO', VV-VV' is now associated to each motor MO-MO', MV-MV', which screws while rotating move a translation block BT associated to said helicoidal screws VV-VV' rotated by motor MV (MV'). A cutting guide GT is associated at the ends of said helicoidal screws VV and VV'.

With this simple system consisting of only two couples of motors MO-MO', MV-MV' and relevant screws a kinematic parallel movement of the cutting guide GT with five liberty degrees is brought about.

According to an other to feature of the invention, in order to reach the six degrees of freedom, which allow the total free spatial movement of the guide GT, a fifth motor MGT is added which puts in rotation according to arrow R (without translation) said cutting guide that now reaches the desired six freedom degrees.

Up to now the systems of automated movements were represented as comprised between or selected from the group consisting of the anthromorphous system of the ABB Company (which by the way has had no users in the operative practice) and the system known under the name Galileo of the PI System AG which comprised only two motors and allowed the movement with only two freedom degrees.

With the system according to the invention we succeed, with the simple addition of only three (compact, cheap and reliable) micro-motors to surprisingly realize the total automation of the cutting guide reproducing thereby, with the maximal precision, the execution of the intervention virtual planning. Therefore the system of the present invention is characterized by a parallel kinematics i.e. a linear movement along at least four axes which are two by two parallel, with the addition of a further movement of sole rotation centered on the operation means, so bringing about a total movement with six freedom degrees.

Said system comprises therefore a first couple of motors each acting on a helicoidal screw, which carry out the linear movement at parallel kinematics in a first plane; - a second couple of motors each acting on a helicoidal screw, which realize the linear movement with a parallel kinematics in a second plane perpendicular to said first plane; and a fifth motor which carries out the rotatory movement of the cutting guide around its own axis.

According to the invention, the first couple of motors MO-MO' is not mobile because it is rigidly fixed to the limb to be operated whereas the second couple of motors MV-MV' is free to linearly translate in two perpendicualr directions. In said system of the invention is present a double driving block BT-BT', each one of which moves relatively to the relevant motor MO-MO' stationary during the block rotation, and represents the stationary part in respect to the mobile motors MV-MV'.

Therefore each driving block is animated with (i.e. is put in) a linear movement by one of the stationary motors and on its turn, animates with (i.e. puts in) a linear movement the mobile motor MV-MV' as well as the fifth motor MGT.

The system according to the invention is preferably embodied in form of a robot.

To simplify its structure and working, the invention has been described with particular reference to the anchorage system according to Applicant's Patent Application N° MI2002A 002218. However the invention is not restricted to said anchorage system and obviously comprises all the changes, substitutions, variants and the like which, being in the reach of the mean technician of this Art, fall naturally within the scope and spirit of the present invention.

## Claims

1. System of motorized guide for operation instrumentations especially for orthopedic surgery in particular to knee surgery, comprising a device of true intervention such as a guided cutting means, and means for its movement substantially (up to-day) not automated, **characterized by** a device of parallel kinematics namely of linear movement along at least four axes parallel two by two, with the addition of a further movement of sole rotation centered on said intervention device, whereby a total movement with six freedom degrees is brought about.

2. System according to claim 1, charcterized in that it comprises: - a first couple of motors (MO-MO') each acting on a helicoidal screw carrying out the linear movement of parallel kinematics in a first plane; - a second couple of motors (MV-MV') each acting on a helicoidal screw bringing about the linear movement of parallel kinematics in a second plane perpendicular to said first plane; and - a fifth motor (MGT) carrying out the rotatory movement of said cutting guide around its own axis.

3. System according to claim 2, in which the first couple of motors (MO-MO') is stationary as it is rigidly fixed to the limb to be operated whereas the second couple of motors (MV-MV') is free to linearly translate in a direction perpendicular to the plane of the first motor couple (MO-MO').

4. System according to at least one of the above claims, **characterized by** a double driving block (BT-BT') each one of which moves in respect to its relevant stationary motor (MO-MO') during the rotation of said same motor, and forms the stationary part relative to the mobile motors (MV-MV')

5. System according to claim 4, in which each driving block (BT-BT) is imparted (is put in) a linear movement by one of the stationary motors (MO-MO'), and, on its turn, imparts (puts in) a linear movement to the mobile motor (MV-MV') as well as to the fifth motor (MGT).

6. System according to at least one of the above claims, wherein the therein recited devices and means are compacted in the form of a robot.
